# EUROPEAN PATENT SPECIFICATION

(11) **EP 2 019 118 B1**
(45) Date of publication and mention of the grant of the patent: **07.11.2012**
(21) Application number: 07765833.4
(22) Date of filing: 25.04.2007
(51) Int. Cl.: C07K 14/575, C07K 17/08, A61K 38/22, A61P 29/00, A61P 35/00

(54) **METHOD FOR SYNTHESIZING THYMOSINS**
VERFAHREN ZUR SYNTHETISIERUNG VON THYMOSINEN
PROCÉDÉ DE SYNTHÈSE DE THYMOSINES

(30) Priority: 10.05.2006 ES 200601197
(43) Date of publication of application: 28.01.2009
(73) Proprietor: BCN Peptides, S.A., Barcelona (ES)
(72) Inventor: FERNANDEZ CARNEADO, Jimena, 08950 Esplugues de Llobregat (ES); PONSATI OBIOLS, Berta, 08005 Barcelona (ES); CLEMENTE RODRIGUEZ, Javier, 08901 L'Hospitalet de Llobregat (ES); RUBIRES FERRER, Raimon, 08720 Vilafranca del Penedés (ES); PAVON FERNANDEZ, Sergi, 08224 Terrassa (ES)
(74) Representative: Carvajal y Urquijo, Isabel
(86) International application number: PCT/ES2007/000246
(87) International publication number: WO 2007/132037

(56) References cited:
- WO-A1-93/05806
- WO-A2-99/49883
- WO-A2-2006/117227
- ES-A1- 2 119 581
- ES-T3- 2 103 966
- ES-T3- 2 192 839
- Anonymous: "Novabiochem innovations 3/04", MERCK, 30 September 2004 (2004-09-30), XP002647091, Retrieved from the Internet: URL:www.merck-chemicals.com/showBrochure?i d=200907.213 [retrieved on 2004-06-26]
- JANET CALDARELLA ET AL: "Thymosin ae,: A peptide related to thymosin a1 isolated from calf thymosin fraction 5", PROCEEDINGS OF THE NATIONAL ACADEMY OF SCIENCES, vol. 80, 1 December 1983 (1983-12-01), pages 7424-7427, XP007918872, NATIONAL ACADEMY OF SCIENCES, WASHINGTON, DC; US ISSN: 0027-8424
- HUTCHINSON L M ET AL: "Development of a sensitive and specific enzyme-linked immunosorbent assay for thymosin beta15, a urinary biomarker of human prostate cancer", CLINICAL BIOCHEMISTRY, vol. 38, no. 6, 1 June 2005 (2005-06-01), pages 558-571, XP004882648, ELSEVIER INC, US ISSN: 0009-9120, DOI: 10.1016/J.CLINBIOCHEM.2005.01.015
- ZIKOS C ET AL: "COMPARATIVE EVALUATION OF FOUR TRITYL-TYPE AMIDOMETHYL POLYSTYRENE RESINS IN FMOC SOLID PHASE PEPTIDE SYNTHESIS", JOURNAL OF PEPTIDE SCIENCE, vol. 9, no. 7, 1 July 2003 (2003-07-01), pages 419-429, XP009058375, JOHN WILEY AND SONS LTD, GB ISSN: 1075-2617, DOI: 10.1002/PSC.454
- VASSILIKI KOUTRAFOURI ET AL: "Synthesis and angiogenetic activity in the chick chorioallantoic membrane model of thymosin beta-15", PEPTIDES, vol. 24, 1 January 2003 (2003-01-01), pages 107-115, XP007918871, ELSEVIER, AMSTERDAM ISSN: 0196-9781
- TORSTEN WÖHR ET AL: "Pseudo-Prolines as a Solubilizing, Structure-Disrupting Protection Technique in Peptide Synthesis", JOURNAL OF THE AMERICAN CHEMICAL SOCIETY, vol. 118, no. 39, 2 October 1996 (1996-10-02), pages 9218-9227, XP002447320, AMERICAN CHEMICAL SOCIETY, WASHINGTON, DC; US ISSN: 0002-7863, DOI: 10.1021/JA961509Q
- [Online] Retrieved from the Internet: <URL:http://www.emdbiosciences.SharedImages /Novabiochem/03_04_innovE.pdf>

## Description

### Field of the Invention

This invention is comprised within the field of biomedical chemistry. The invention particularly relates to a new method for chemically synthesizing thymosins by means of solid-phase peptide synthesis with high yield and purity. The method is based on incorporating pseudoproline derivatives in key positions of the thymosin sequences. The pseudoproline oxazolidine cycle prevents the structuring of the growing peptide chain anchored to the resin, preventing the aggregation of the peptidyl resin and the collapse of the synthesis during the elongation of the peptide sequence.

### Background of the Invention

Thymosins are a family of biochemically and functionally different polypeptides with important physiological functions. Described for the first time in 1966 and originally isolated in the thymus, they have been subsequently identified in several tissues and cells *[*Goldstein, A.L., Slater, F.D., White, A., Preparation and partial purification of a thymic lymphocytopoietic factor, Proc. Natl. Acad. Sci. USA 1966, 56 (3), 1010-1017*].*

There are different families of thymosins carrying out different functions, for example, α-thymosins, having nuclear localization and involved in DNA replication and/or transcription, and β-thymosins, which are located in the cytoplasm and show great affinity for G-actin in processes involved in cell motility. α- and β-thymosins have an important role in the regulation of immune response, vascular biology and cancer pathogenesis, having a great therapeutic potential in addition to their application in diagnosis as molecular markers *[*Goldstein A.L.; Badamchian M., Thymosins: chemistry and biological properties in health and disease, Expert Opin. Biol. Therapy, 4 (4), 559-573*]*.

The subfamily of α-thymosins is formed by parathymosin a, prothymosin α, thymosin α1 and thymosin α11. The most important one is thymosin α1 or thymalfasin, originally isolated from thymus tissue, and it is a peptide with 28 amino acids, acylated in the N-terminal position and with an acid group at its C-terminal end, derived from the prothymosin α1 precursor with 111 amino acids. Due to its immunoregulating properties, thymosin α1 has been used for treating diseases in which the immune system is affected; mainly in hepatitis B, C and liver carcinoma. Chronic hepatitis B is a disease related to an immune system dysfunction and entails a high risk of suffering from cirrhosis and hepatocellular carcinoma. Thymosin α1 acts as an immune system modulator activating the production of NK cells in several animal and human models and increasing the production of CD3, CD4 and CD8 cells in patients with chronic hepatitis B and cancer. It also activates the production of Th1 cytokines, which are relates to a strong antiviral immune response.

A number of *in vitro* cell models support the therapeutic potential of thymosin α1. The treatment with thymosin α1 reduces the HIV-1 and parainfluenza virus replication in primary cell cultures *[*Colledge, D., Whang, Y. Tuthill C.; Locarnini, S. Antiviral effects of thymosin alpha 1 versus leukocytic interferon in primary cultures of duck hepatocytes persistently infected with duck HBV in vitro, Second international conference on therapies for viral hepatitis, 1997*;* Kona, Hawai; Palamara, A. Bue M., Savini, P., Thymosin alpha 1 inhibits Sendai virus replication: involvement of intracellular redox state, 6th International Expert Forum of Immunotherapy and Gene Therapy; May 1998, Florence, Ital*y].* It further reduces oxidative stress, increasing the levels of intracellular glutathione the low concentration of which in HIV patients is related to a low survival rate *[*Giuliani, C., Napolitano, G., Mastino, A., Thymosin alpha-1 regulates MHC class I expression in FRTL-5 cells at transcriptional level, Eur. J. Immunol. 2000, 30, 778-786*]*. Thymosin α1 increases MHC class 1 expression levels, maintaining the capacity of the immune system to response to infectious agents *[*Herzenberg, L.A.; De Rosa, S.C.; Dubs, J.G., Glutathione deficiency is associated with impaired survival in HIV disease, Proc. Natl. Acad. Sci. USA, 1997, 94, 1967-1972]

In animal infection models, the therapeutic potential of thymosin α1 has been shown in mice infected with several pathogens (Listeria monocitogenes, Candida albicans, Pseudomonas aeruginosa and Serratia marcescens) and in mice immunosuppressed with 5-fluorouracil *[*Ishitsuka, H; Umeda, Y. Nakamura,J. Yagi, Y. Protective activity of thymosin against opportunistic infections in animal models. Cancer Immunol. Immunother. 1983, 14, 145-150*].* Thus, in studies with animal models of chronic hepatitis B (woodchuck hepatitis virus) in which the animals followed a treatment with thymosin α1, the serum virus concentration was reduced by 99% *[*Gerin, J.L., Korba, B.E., Cote P.J., Tennant, B.C., A preliminary report of a controlled study of thymosin alpha 1 in the woodchuck model of hepanavirus infection. In: Block T., ed. Innovations in antiviral development and the detection of virus infection. Philadelphia, Pa: Jefferson Medical; 1992,121-123*;* Tennant, B.V., Korba, B.E., Baldwin B.H., Goddard, L.A., Hornbuckle W.E., Cote, P.J., Treatment of chronic woodchuck hepatitis virus infection with thymosin alpha-1 (TA1). Antiviral Res. 1993; 20 (suppl 1), 1634*],* said treatment reducing the risk of death by hepatocellular carcinoma. Thymosin α1 also increases the survival of adult or immunosuppressed animals infected with herpes simplex virus, influenza virus or *Candida albicans [*Effros, R.B., Casillas, A., Wadlford, R.L., The effect of thymosin alpha 1 on immunity to influenza in aged mice. Aging: immunology and infectious disease. Vol 1. New York, N. Y.: Mary Ann Liebert, Inc; 1988; 31-40*;* Bistoni, F., Marconi, P., Frati, L., Bonmassar, E., Garaci, E. Increase of mouse resistance to candida albicans infection by thymosin alpha1. Infect. Immun. 1982, 36, 609-614*].*

The synergistic effect of thymosin α1 with other cytokines in the treatment of hepatitis C has been shown in tests with mice infected with the influenza A virus treated with a combination of thymosin α1, interferon IFNα/β and the antiviral agent amantadine. The combination of the three compounds substantially improves the survival percentage *[D'*Agostini C., Palamara, A.T., Favalli, C. Efficacy of combination therapy with amantadine, thymosin alpha 1 and alpha/beta interferon in mice infected with influenza A virus. Int. J. Immunopharmacol. 1996, 16, 95-102*].*

A study in which the effect of the treatment with thymosin α1 or interferon IFN α-2b was comparatively studied in difficult-to-treat patients with a hepatitis B virus mutant shows almost twice the efficacy in eliminating the virus with thymosin α1. In contrast to IFNα-2b, thymosin α1 is better tolerated and furthermore does not present significant side-effects in immunosuppressed people *[*Andreone, P., Cursaro, C., Gramenzi, A., A randomized controlled trial of thymosin alpha 1 versus interferon alpha treatment in patients with hepatitis B antigen antibody and hepatitis B virus DNA, positive chronic hepatitis B, Hepatology, 1996, 24, 774-777*]. While interferon causes a* fast response during the treatment, resistant virus strains cause the relapse of the patient, thymosin α1 has a more delayed effect related to a long-term improvement of the immune system *[*Garaci, E., Milanese, G., Vella, S., A randomized controlled study for the evaluation of the activity of a triple combination of zidovudine, thymosin alpha 1 and interferon alpha in HIV-infected individuals with CD4 counts between 200 and 500 cells/mm3, Antiviral Therapy, 1998, 3, 103-11*].*

In animal cancer models, α-thymosin has demonstrated its efficacy in the prevention of intestinal cancer *[*Moody, T.W., Leyton, J., Zia, F., Tuthill, C., Badamchian, M. Goldstein, A.L., Thymosin alpha1 is chemopreventive for lung adenoma formation in A/J mice, Cancer Lett. 2000, 155, 121-127*]*, breast cancer, reducing the incidence of cancer development as a result of stimulating the immune system in rats *[*Moody, T.W, Tuthill, C., Badamchian, M., Goldstein, A.L., Thymosin alpha1 inhibits mammary carcinogenesis in Fisher rats, Peptides, 2002, 23 (5), 1011-1014*]* and glioblastoma *[Patent* WO03049697*, Thymosin-alpha1 is used* as *an adjuvant in combination with carmustine (BCNU)* as an *effective treatment for malignant glioblastoma,* Wands, J.R. Of the Monte S., Rhode *Island Hospital* (US), *30-06-2005].* The combined therapy of interferon IFN α-2b, thymosin α1 and cyclophosphamide has given good results, reducing the size of melanoma in mice *[*Pica, F., Fraschetti, M., Matteucci, C., Tuthill, C., Rasi, G., High doses of thymosin alpha 1 enhance the anti-tumor efficacy of combination chemo-immunotherapy for murine B16 melanoma, Anticancer Res. 1998, 18, 3571-3578*].*

thymosin α1 and as potent as it in Candida albicans infections have been isolated from thymosin fraction 5 preparations: des-(25-28)- thymosin α1 and thymosin α11 *[*Thymosin α11: a peptide related to thymosin α1 isolated from calf thymosin fraction 5, Calderella, J., Goodall, G.J., Felix, A.M., Heimer, E.P., Salvin, S.B., Horecker, B.L., Proc. Natl. Acad. Sci. USA, 80, 7424-742*].*

The second subfamily of thymosins are β-thymosins forming a family of proteins (thymosins β-4, β-8, β-9, β-10, β-11, β-12, β-15) with a high homology in their peptide sequence (see Table 1).
Table 1 shows the thymosin sequences.

**TABLE 1**

| **SEQUENCES** | **PEPTIDE SEQUENCES** | **Abbreviated Name** |
|---|---|---|
| SEQ.ID.N.1 | Ac-SDAAVDTSSEITTKDLKEKKEVVEEAEN-OH 28AA | Thymosin α1 |
| SEQ.ID.N.2 | Ac-SDAAVDTSSEITTKDLKEKKEVVE-OH 24AA | Des-(25-28)-Thymosin α1 |
| SEQ.ID.N.3 | Ac-SDAAVDTSSEITTKDLKEKKEWEEAENGREAPAN-OH 35 AA | Thymosin α11 |
| SEQ.ID.N.4 | Ac-SDKPDMAEIEKFDKSKLKKTETQEKNPLPSKETIEQEKQAGES-OH...43AA | Thymosin β4 |
| SEQ.ID.N.5 | Ac-SDKPDMAEIEKFDKAKLKKTETQEKNPLPSKETIENEKNTSGES-OH 44AA | Thymosin β4^{xen} |
| SEQ.ID.N.6 | Ac-ADKPDLGEINSFDKAKLKKTETQEKNTLPTKETIEQEKQ-OH 39AA | Thymosin β8 |
| SEQ.ID.N.7 | Ac-ADKPDLGEINSFDKAKLKKTETQEKNTLPTKETIEQEKQAK-OH 41AA | Thymosin β9 |
| SEQ.ID.N.8 | Ac-ADKPDMGEIASFDKAKLKKTETQEKNTLPTKETIEQEKRSEIS-OH 43AA | Thymosin β10 |
| SEQ.ID.N.9 | Ac-SDKPNLEEVASFDKTKLKKTETQEKNPLPTKETIEQEKQAS-OH 41AA | Thymosin β11 |
| SEQ.ID.N.10 | Ac-SDKPDLAEVSNFDKTKLKKTETQEKMPLPTKETIEQEKQATA-OH 42AA | Thymosin β12 |
| SEQ.ID.N.11 | H₂N-SDKPDLSEVETFDKSKLKKTNTEEKNTLPSKETIQQEKEYNQRS-OH 44AA | Thymosin β15 |

Thymosin β4 contains 43 amino acids and is expressed in embryonic development stages corresponding to heart development. This discovery, carried out in preclinical studies with animals, has emphasized the potential of thymosin β4 for treating patients which have a suffered a myocardial infarction. In preclinical studies with mice in which heart attacks were induced, the treatment with the thymosin β4 protein caused the differentiation of endothelial cells and stimulated the migration of cardiac cells, improving the capacity of said cells *[*Bock-Marquette, I., Saxena, A., White, M.D., DiMaio, J.M., Srivastava, D., Thymosin β4 activates integrin-linked kinase and promotes cardiac cell migration, survival and cardiac repair, Nature, 2004, 432, 466-472*]*

Thymosin β4 further regulates a series of inflammatory chemokines and cytokines, it controls the function of the actin protein, binding to it, and induces the production of laminin-5, a protein necessary for the correct adhesion of some types of mammalian cells and an important component in the wound healing process, thus facilitating reepithelization and inducing collagen deposition *[*Bubb, M.R., Thymosin beta 4 interactions, Vitam. Horm. 2003, 66, 297-316*].*

Other possible therapeutic applications of thymosin β4 include the increase of hair growth by activating hair follicle stem cells *[Patent* WO/063775 A2*, Methods and compositions for the promotion of hair growth utilizing actin binding peptides].* Also for the treatment of several types of chronic dermatological indications such as chronic venous stasis ulcers, chronic pressure ulcers, epidermolysis bullosa, a group of hereditary disorders characterized by the formation of blisters in response to mechanical trauma and treatment of corneal injuries. The LKKTETQ fragment of thymosin β4, the actin binding domain, facilitates curing dermal wounds in adult animals *[*Philp D., Huff, D., Song Gho, Y.; Hannappel, E., Kleinman, T., The actin binding site on thymosin β4 promotes angiogenesis, FASEB J., 17, 2103-2105*].*

Thymosin β15 is a peptide with 44 amino acids overexpressed in prostate cancer, in which it favors tumor dissemination outside the prostate gland. This peptide is being developed as a biomarker for diagnosing said cancer, for discriminating between benign and malignant prostate tumors and for treating them suitably *[*Hutchinson, L. M., Chang, E.L., Becker, C. M., Ushiyama, N., Behonick, D., Shih, M. C., DeWolf, W.C., Gaston, S.M., Zetter, B.R., Development of a sensitive and specific enzyme-linked immunoassay for thymosin beta 15, a urinary biomarker of human prostate cancer, Clin. Biochem. 2005, 38 (6), 558-571*].* Thymosin β15 has also been patented for its use in wound regeneration and healing and can be applied for this indicated in the near future *[Patent* CN1579539*, Thymic hormone beta 15 to promote wound healing and generation of blood, Gan Chunyu (CN)].*

Thymosin β10 is formed by 43 amino acids and is overexpressed in embryonic and tumor cells as colon adenocarcinomas *[*Vasile, E., Tomita, Y., Brown, L.F., Kocher, O., Dvorak, H.F., Differential expression of thymosin ß-10 by early passage and senescent vascular endothelium is modulated by VPF/VEGF: evidence for senescent endothelial cells in vivo at sites of atherosclerosis, FASEB J., 2001,15,458-466*].* Thymosin β10 has a great homology with thymosin β4 since both of them bind to actin and inhibit its polymerization *[*Yu, F.X, Lin, S.C., Morrison-Bogorad, M., Atkinson, M.A., Yin, H.L., Thymosin beta 10 and thymosin beta 4 are both actin monomer sequestering proteins, J. Biol. Chem., 1993, 268 (1), 502-509*].* Recent results related to the binding of the thymosin β10 to actin and the activation of the apoptotic process in ovarian cancer cells is demonstrating the potential of thymosin β10 and its derivatives in a new therapy for treating ovarian cancer *[*Rho, S.B., Lee, K. W., Chun, T., Lee, S-H., Park, K., Lee, J-H., The identification of apoptosis-related residues in human thymosin β-10 by mutational analysis and computational modelling, J. Biol. Chem., 280 (40), 34003-34007*].*

None of the β-thymosins with therapeutic potential has reached clinical phases for the moment, although the use of the thymosin β4 and the thymosin β15 for treating patients who have suffered from a myocardial infarction in regeneration of the damaged tissue will soon be submitted to the FDA for its application in clinical phase 1.

Among the different β-thymosins (see Table 1), thymosin β4, thymosin β9 and - thymosin β10 are found in mammals and in contrast, thymosin β4^{Xen} has been identified in amphibians (Xenopus) *[*Voelter, W., Kaiser, T., Hannappel, E., Echner, H., Synthesis of thymosin β4xen and its comparison with the natural tritetraconpeptide, J. Prakt. Chem., 1999, 341(1), 47-51*]* and β11 and β12 in trout (Salmo gairdneri) *[*Yialouiris, P.P., Coles, B., Tsitsiloni, O., Schmid, B., Howell, S., Aitken, A., Voelter, W., Haritos, A.A., The complete sequences of trout (Salmo gairdneri) thymosin β11 and its homologue thymosin β12, Biochem.J., 1992, 283, 385-389*].*

Thymosin β8 contains 39 amino acids and a high degree of homology (79%) with thymosin β4. Thymosin β9 has 41 amino acids and differs from thymosin β9 in additional dipeptide in the C-terminal region, having 82% homology with thymosin β4. Thymosin β11 with 42 amino acids has 78% homology in relation to thymosin β4. Thymosin β12 with 43 amino acids is 79% homologous to thymosin β4 and 84% to thymosin β11 (see Table 1).

The first patented thymosin synthesis method was for obtaining thymosin α1. This method proposed the convergent synthesis in solution of two fragments with fifteen (15) and thirteen amino acids (13) which had previously been synthesized in solid phase with the Boc/Bzl strategy *[Patent* US4148788*, Synthesis of thymosin alpha 1, Hoffmann La Roche, 1979].* This method was subsequently implemented in a strategy carried out in solution in which seven (7) fragments were used *[Patent* US4504415*, Intermediates for thymosin alpha 1 and desacetyl thymosin alpha 1, Hoffmann La Roche, 1985].*

In these previously described methods, the strategy used to synthesize fragments in solid phase was Boc/Bzl. Another option that was also used was the use of a temporary N-terminal position protecting group, the so-called Ddz *[*Nguyen, T.H., Heinzel, W., Birr, C., Ddz, a highly efficient protecting group in large scale syntheses of thymosin-α1 related peptides, Pept., Proc. Eur. Pept. Symp., 19th, 1987*].*

Another method which has also been described is that of the complete synthesis of thymosin α1 according to a Boc/Bzl linear solid-phase strategy on an MBHA resin, with the use of HBr and subsequently bromotrimethylsilane to cleave the peptide from the resin *[*Wang, S., Wang, B.S.H., Hughes, J.L., Leopold, E.J., Wu, C. R., Tam, P., Cleavage and deprotection of peptides on MBHA-resin with hydrogen bromide, Int.J.Pept. Prot. Res., 1992, 40(3-4), 344-349*;* Hughes, J.L., Leopold, E.J., Cleavage and deprotection of peptides from MBHA-resin with bromotrimethylsilane, Tetrahedron Lett., 1993, 34(48), 7713-7716*].*

In addition, there is also another patented method for the convergent synthesis in solution of fragments previously obtained in solid phase which uses temporary Moz protection instead of Boc protection, improving the yields and the purity of the end product *[Patent* US5856440*, Solid phase process for synthesizing thymosin alpha1, Alpha 1 Biomedicals Inc., 1999].*

However, the method using the orthogonal Fmoc/tBu strategy is currently the most popularly accepted one due to the elimination of the work steps with TFA for eliminating the temporary N-terminal protecting group and HF for cleaving the peptide from the resin. With the Fmoc/tBu strategy, a proposed method for synthesizing thymosin α1 includes the use of protected side chains and the coupling in solution of two fragments (1-10) and (11-28) with DCC/HOBT with a 30% yield for the last 4 steps *[*Felix, A.M., Heimer, E.P., Wang, C.T., Lambros, T.J., Swistok, J., Roszkowski, M., Ahmad, M., Confalone, D., Scott, J.W, Synthesis of thymosin α1 by fragment condensation using tert-butyl side chain protection, Int. J. Pept. Prot. Res., 1985, 26 (2), 130-148*].* The previously described coupling step has been optimized until obtaining a 44% yield in TFE. Although the coupling yield in CHCl₃ is 49%, the purity of the crude product obtained in the latter is worse *[*Felix, A.M., Wang, C. T,, Lambros, T.J., Coupling of large protected peptide fragments in trifluoroethanol: synthesis of thymosin α1 Pept.: Struct. Funct., Proc. Am. Pept. Symp., 9th, 1985*].* The step-by-step linear solid-phase strategy with Fmoc/tBu protectors has also been described using a p-benzyloxycarbonyl resin *[*Echner, H., Voelter, W., A new synthesis of thymosin α1, Liebigs Annalen der Chimie, 1988, 11, 1095-1097*].*

Two linear strategies have been described for obtaining thymosin β4 in solid phase: a Boc/Bzl strategy *[*Low, T.L.K., Wang, S.S., Goldstein, A.L., Solid-phase synthesis of thymosin β4: chemical and biological characterization of the synthetic peptide, Biochemistry, 1983, 22, 733-740*]* and a Fmoc/tBu strategy *[*Zikos, C., Livaniou, E., Leondiadis, L., Ferderigos, N., Ithakissios, D.S., Evangelatos, G.P., Comparative evaluation of four trityl-type amidomethyl polystyrene resins in Fmoc solid phase peptide synthesis, J. Pept. Sci, 2003, 9(7), 419-429*]*. In the latter strategy, using labile chloro-trityl-type resins, thymosin β4 is obtained with a 30% yield, better than that obtained with the Boc/Bzl strategy (5-12%) but which can be improved from the industrial point of view (see Table 2, showing the comparison of yields obtained in the synthesis of beta-thymosins).

**Table 2**

| Product | Synthesis strategy | Yield product | Purity of the crude pruduct (HPLC) | Reference | Comments | Resin and synthesis conditions |
|---|---|---|---|---|---|---|
| Thymosin β10 | Fmoc/tBu | **40%** | n.i. | *Leondiadis, L. et al., J. Chem. Soc., Perkin Trans.1. 1996, 971-975* | - prolonged couplings overnight including an acetylation for: | - bromo-4-cyanotrityl resin (0.56 mmol/g) |
| | | | | | step K3, A15, K16, L28, T30, T33, | - 10 eq. Fmoc-AA-OH, 10 eq. DIPCDI, 10 eq. HOBT |
| Thymosin β15 | Fmoc/tBu | **45%** | n.i. | Koutrafouri, V. et al., Peptides 2003, 24, 107-115 | - long and incomplete couplings including an acetylation step for: P4, K16, 134: | - 4-cyano-4'-carboxymethoxytrityl resin |
| | | | | | | - Fmoc-AA, HOBT, DIPCDI |
| Thymosin β4^{Xen} | Fmoc/tBu | n.i. | **48-53%** | Voelter, W et al., J. Prakt. Chem., 1999, 341, 47-51 | - double couplings for all the residues | HMP resin (0.5 mmol/g) |
| | | | | | | - 4 eq. Fmoc-AA, HOBT, DCC |
| Thymosin β4 | Boc/Bzl | **5-12%** | n.i. | Low, T. L. K., et al., Biochemistry, 1983, 22, 733-740 | - double couplings | - Boc-Ser(Bzl)-PAM resin (0.11 mmol/g) |
| | | | | | | - 10 eq. Boc-AA, DCC |
| Thymosin β4 | Fmoc/tBu | **30%** | **60%** | Zikos, C., et al., J.Pept.Sci, 2003, 9(7), 419-429 | - double couplings for: | - amidomethyltrityl resin |
| | | | | | S1, D2, K3, P4, D5, 19, K14, K16, K25, I28, S30 | - 5 eq Fmoc-AA, HOBT, DIPCDI |
| Thymosin β4 | Fmoc/tBu | n.i. | *n.i.* | *BCN Peptides solid-phase convergent synthesis process* | Synthesis of two fragments (1-27)+(28-43)-resin | - 2-chlorotrityl resin (0.85 mmol/g) |
| | | | | | | - 3 eq Fmoc-AA, HOBT and DIPCDI |
| Thymosin β4 | Fmoc/tBu | **30%** | **< 30%** | *BCN Peptides conventional linear synthesis process* | - double couplings for: 134, T33, L28, N26, K16, S15, D13, E10, 19, E8, M6, D5 | - 2-chlorotrityl resin (0.85 mmol/g) |
| | | | | | | - 3 eq. Fmoc-AA, HOBT and DIPCDI, |
| Thymosin β4 | Fmoc/tBu | **50 %** | > **80%** | *BCN Peptides linear synthesis process with pseudoprolines* | - double couplings for: N26 | - 2-chlorotrityl (0.85 mmol/g) |
| | | | | | | - 3 eq Fmoc-AA, HOBT and DIPCDI, |
| | | | | | | - 2.5 eq. pseudoproline dipeptides |

One thymosin β4 variant, in which the Ser15, Ala40 and Ser41 positions of thymosin β4 are substituted with Ala, Thr and Ser respectively, is thymosin β4^{Xen} which has already been synthesized according to a linear Fmoc/tBu strategy and from which a 48-53% purity of the crude product has been obtained *[*Voelter, W , Kaiser, T., Hannapel, E., Echner, H, Synthesis of thymosin β4xen and its comparison with the natural tritetraconpeptide, J. Prakt. Chem., 1999, 341, 1, 47-51*].*

The solid-phase synthesis of thymosin β10 has already been described following a linear synthesis strategy with a bromo-4-cyanotrityl resin. The synthesis method includes prolonged couplings overnight, recouplings and acetylations in several positions *[*Leondiadis, L., Vassiliadou, I., Zikos, C., Ferderigos, N., Livaniou, E., Ithakissios, D.S., Evangelatos, G.P., Solid-phase synthesis of thymosin β10 using a p-cyanotrityl resin. Chemical characterization and immunochemical control of the synthetic peptide, J. Chem. Soc., Perkin Trans. 1, 1996, 971-975*].* The also described synthesis of thymosin β15 is obtained by means of a similar strategy, i.e. a linear synthesis based on the Fmoc/tBu strategy. This synthesis leads to the thymosin β15 product but also involves long and incomplete couplings including an acetylation step, the purity of the crude product obtained decreasing *[*Koutrafouri, V., Leondiadis, L., Ferderigos, N., Avgoustakis, K., Livaniou, E., Evangelatos, G.P., Ithakissios, D.S. Synthesis and angiogenetic activity in the chick chorioallantoic membrane model of thymosin beta-15, Peptides, 2003, 24, 107-115*].* The overall yields obtained are not low (40% of thymosin β10) and (45% of thymosin β15) although the synthesis method needs to be optimized for large scale production.

In solid-phase peptide synthesis, the hydrophobic interactions of the protected growing peptide chain gives rise to low coupling yields, incomplete deprotections and to obtaining by-products. The use of pseudoprolines recently introduced into the synthesis of long peptides is a substantial improvement for the state of the art in peptides with a great tendency to be structured during the elongation of the chain.

Pseudoprolines are cyclic Ser, Thr or Cys derivatives which are useful as protecting groups for these amino acids (Formula I) and facilitate synthesizing difficult sequences, interrupting the structuring of the growing peptide chain still anchored to the resin and preventing the aggregation of the peptidyl resin and the collapse of the synthesis *[*Wöhr, T., Wahl, F., Nefzi, A., Rohwedder, B., Sato, T., Sun, X., Mutter, M., Pseudoprolines as a solubilizing, structure-disrupting protection technique in peptide synthesis, J. Am. Chem. Soc., 1996, 118, 9218-9227*].* Pseudoprolines introduce bends in the backbone of the peptide chain, their use being especially interesting in the synthesis of hydrophobic sequences or long peptides *[*Abedini, A., Raleigh, D.P., Incorporation of pseudoproline derivatives allows the facile synthesis of human IAPP, a highly amyloidogenic and aggregation-prone polypeptide, Organic Lett., 2005, 7(4), 693-696*;* White, P., Keyte, J. W., Bailey, K., Bloomberg, G., Expediting the Fmoc solid phase synthesis of long peptides through the application of dimethyloxazolidine dipeptides, J. Peptide Sci., 2004, 10, 18-26*; von* Eggelkraut-Gottanka, R., Machova, Z., Grouzmann, E., Beck-Sickinger, A.G., Semisynthesis and characterization of the first analogues of pro-neuropeptide Y, ChemBioChem, 2003, 4, 425-433*].*

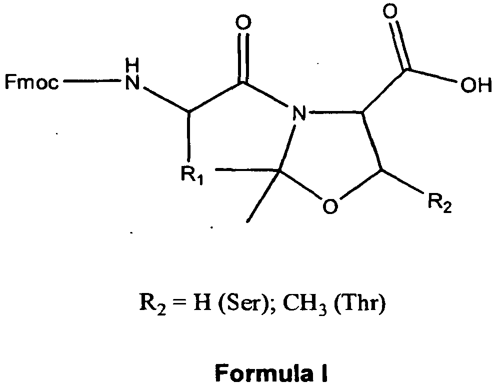

Once the synthesis has ended, the sequence and native structure of the peptide are regenerated by means of the final acid treatment involving the cleavage and deprotection of the side chains and the opening of the oxazolidine cycle (Scheme 1).

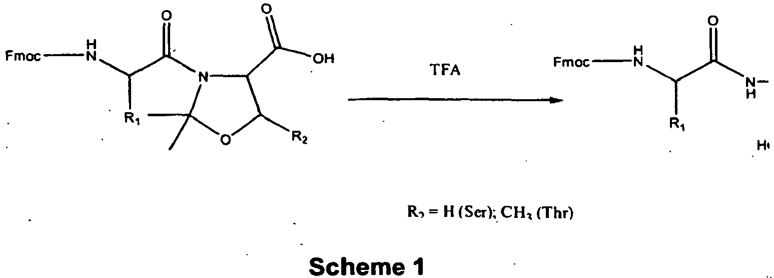

Table 3 shows the thymosin sequences; the Ser and Thr which could be substituted with pseudoprolines are underlined.

As has been seen above, although the synthesis of long peptides in the academic field has already been described in the state of the art, pseudoprolines have not been applied on an industrial scale or for synthesizing thymosins, which supports the inventive novelty and capacity and step of the present invention.

**TABLE 3**

| PEPTIDE SEQUENCES | Abbreviated name |
|---|---|
| Ac-**S**DAAVDT**SS**EI**TT**KDLKEKKEVVEEAEN-OH 28AA | Thymosin α1 |
| Ac-**S**DAAVD**TSS**EITTKDLKEKKEVVE-OH 24AA | Des-(25-28)-Thymosin α1 |
| Ac-**S**DAAVD**TSS**EI**TT**KDLKEKKEVVEEAENGREAPAN-OH 35 AA | Thymosin α11 |
| Ac-**S**DKPDMAEIEKFDK**S**KLKK**T**E**T**QEKNPLP**S**KE**T**IEQEKQAGE**S**-OH 43AA | Thymosin β4 |
| Ac-**S**DKPDMAEIEKFDKAKLKK**T**E**T**QEKNPLP**S**KE**T**IENEKNT**S**GE**S**-OH 43AA | Thymosin β4^{xen} |
| Ac-ADKPDLGEIN**S**FDKAKLKK**T**E**T**QEKN**T**LP**T**KE**T**IEGIEKQ-OH 39AA | Thymosin β8 |
| Ac-ADKPDLGEIN**S**FDKAKLKK**T**E**T**QEKNTLP**T**KE**T**IEQEKQAK-OH 41AA | Thymosin β9 |
| Ac-ADKPDMGEIA**S**FDKAKLKK**T**E**T**QEKN**T**LP**T**KE**T**IEQEKR**S**EI**S**-OH 43AA | Thymosin β10 |
| Ac-**S**DKPNLEEVA**S**FDK**T**KLKK**T**E**T**QEKNPLP**T**KE**T**IEQEKQA**S**-OH 41AA | Thymosin β11 |
| Ac-**S**DKPDLAEV**S**NFDK**T**KLKK**T**E**T**QEKMPLP**T**KE**T**IEQEKQA**T**A-OH 42AA | Thymosin β12 |
| H₂N-**S**DKPDL**S**EVE**T**FDK**S**KLKK**T**N**T**EEKN**T**LP**S**KE**T**IQQEKEYNQR**S**-OH 44AA | Thymosin β15 |

### Description of the Invention

The present invention describes a new synthesis method for industrially preparing and obtaining (a and β) thymosins using pseudoproline dipeptides protected in strategic positions (See Table 4). The method for synthesizing thymosins proposed in the present invention specifically describes a method consisting of the following steps:
a) Linearly synthesizing the peptides in solid phase on resin applying the Fmoc/tBu methodology,
b) using X-Thr or X-Ser, wherein X is any amino acid in pseudoproline dipeptide form in strategic positions of the sequence according to the following guidelines:
   b.1) The insertion of pseudoproline dipeptides is not necessary in the C-terminal or in the N-terminal position,
   b.2) The incorporation of at least one pseudoproline dipeptide in the sequence,
   b.3) The structure destabilizing effect of pseudoprolines maintains its influence in the next 5-6 amino acids,
   b.4) The optimal separation between pseudoproline dipeptides is of 5-6 amino acids.
   b.5) The minimal separation between two pseudoproline dipeptides or a pseudoproline and a proline is preferably of two residues,
   b.6) The synthesis result is optimal if the pseudoproline dipeptide is located in a hydrophobic residue region.
c) Breaking the peptide-resin bond, deprotecting the side chains, opening the pseudoproline cycle and obtaining thymosins by means of treating the peptidyl resin with trifluoroacetic acid,
d) In the event of the existence of the Met amino acid in the sequence, treating for the detertbutylation of the crude product with AcOH prior to the purifying by RP-HPLC.

The basic difference in relation to other already existing synthesis methods is the additional incorporation in the sequence of X-Thr and/or X-Ser in protected pseudoproline dipeptide form which leads to obtaining the end product with very good yields and high purity (see Table 2), the purity of the crude product being greater than 80%, the purity of the pure product being greater than 99% and the overall yield being greater than 50%.

Table 3 shows the thymosin sequences with all the possible positions for incorporating pseudoproline dipeptides.

The object of the present invention was developed because the synthesis of long peptides is a challenge fundamentally due to inefficient couplings and deprotections, derived from the aggregation effects which that are also found in not very long but especially hydrophobic or repetitive sequences.

The present invention intends to solve this technical drawback and to that end, it proposes a synthesis method for industrially obtaining thymosins based on solid-phase peptide synthesis, applying the Fmoc/tBu methodology on a 2-chlorotrityl-type resin, incorporating X-Ser and X-Thr in key positions as pseudoproline dipeptides.

The solid-phase synthesis of thymosins with an acid C-terminal function is carried out from a 2-chlorotrityl chloride resin *[Barlos K*., *Chatzi, O.*, *Gatos, D., Stavropoulos, G, 2-Chlorotrityl chloride resin, Int. J. Peptide Protein Res., 1991 (37)*, *513-520].* This resin facilitates incorporating the first amino acid and minimizes the secondary racemization reaction. This resin further reduces the risk of diketopiperazine formation in the basic treatment with 20% piperidine in DMF after incorporating the second amino acid on the resin.

Based on a linear Fmoc/tBu synthesis strategy, as mentioned above, the present invention is characterized in that it incorporates Thr or Ser amino acids, according to the peptide sequence through or by means of adding pseudoproline dipeptides in strategic positions of the peptide sequence.

**Table 4**

| PEPTIDE SEQUENCES | Abbreviated name |
|---|---|
| Ac-SDAAVDTSSE**IT**TKDLKEKKEWEEAEN-OH 28AA | Thymosin α1 |
| Ac-SDAAVDTSSE**IT**TKDLKEKKEWE-OH 24AA | Des-(25-28)-Thymosin α1 |
| Ac-SDAAVDTSSE**ITT**KDLKEKKEWEEAENGREAPAN-OH 35 AA | Thymosin α11 |
| Ac-SDKPDMAEIEKFD**KS**KLKKT**ET**QEKNPLPSK**ET**IEQEKQAGES-OH 43AA | Thymosin β4 |
| Ac-SDKPDMAEIEKFDKAKLK**KT**ETQEKNPLPSK**ET**IENEKNTSGES-OH 43AA | Thymosin β4^{xen} |
| Ac-ADKPDLGE**INS**FDKAKLKKT**ET**QEKNTLPTK**ET**IEQEKO-OH 39AA | Thymosin β8 |
| Ac-ADKPDLGE**INS**FDKAKLKKT**ET**QEKNTLPTK**ET**IEQEKQAK-OH 41AA | Thymosin β9 |
| Ac-ADKPDMGEI**AS**FDKAKLKKT**ET**QEKNTLPTK**ET**IEQEKRSEIS-OH 43AA | Thymosin β10 |
| Ac-SDKPNLEEV**AS**FDKTKLKKT**ET**QEKNPLPTK**ET**IEQEKQAS-OH 41AA | Thymosin β11 |
| Ac-SDKPDLAE**VS**NFDKTKLKKT**ET**QEKMPLPTK**ET**IEQEKQATA-OH 42AA | Thymosin β12 |
| H₂N-SDKPDLSEV**ET**FDKSKLKKT**NT**EEKNTLPSK**ET**IQQEKEYNQRS-OH 44AA | Thymosin β15 |

The present invention is therefore characterized in that it incorporates Thr or Ser amino acids, according to the peptide sequence, by means of adding pseudoproline dipeptides in strategic positions of the peptide sequence according to the following guidelines:
a) The insertion of pseudoproline dipeptides is not necessary in the C-terminal or in the N-terminal position,
b) The incorporation of at least one pseudoproline dipeptide in the sequence,
c) The structure destabilizing effect of pseudoprolines maintains its influence in the next 5-6 amino acids,
d) The optimal separation between pseudoproline dipeptides is of 5-6 amino acids.
e) The minimal separation between two pseudoproline dipeptides or a pseudoproline and a proline is preferably of two residues,
f) The synthesis result is optimal if the pseudoproline dipeptide is located in a hydrophobic residue region.

Obtaining and optimizing a synthesis process for producing thymosins on an industrial level has involved a great effort. This laboratory has unsuccessfully tackled other synthesis strategies for obtaining thymosins. Three different strategies have been studied for obtaining a profitable productive process for producing thymosin β4.

### First strategy by means of solid-phase convergent synthesis:

The synthesis of thymosin β4 has been tackled in a first solid-phase convergent synthesis strategy using two fragments (1-27) and (28-43)-resin. The solid-phase activation and coupling step for the protected fragment 1-27 on the protected 28-43-resin peptidyl resin gave rise to gels that blocked the reaction. The end product could not be identified with the first approach.

### Second strategy by means of linear solid-phase synthesis:

A second approach, the step-by-step linear solid-phase synthesis of thymosin β4 gave rise to a low purity synthesis crude product, unviable for an industrial process for producing thymosin β4 given the difficulty of the purification step and the low overall yield obtained.

### Third strategy by means of linear solid-phase synthesis with the Fmoc/tBu strategy, and the use of pseudoprolines:

The third strategy has involved solving a problem, obtaining thymosins according to a competitive industrial scale process. In the present invention, the use of pseudoprolines has been incorporated in the linear solid-phase synthesis with the Fmoc/tBu strategy in order to obtain a more cost-effective and competitive synthesis process. The results obtained in the comparative synthesis between the linear synthesis strategy of thymosin β4 and the same strategy incorporating pseudoprolines has shown the great advantage involved in the incorporation of pseudoprolines, because pseudoprolines are non-natural amino acids derived from Cys, Ser or Thr with a structure similar to proline. The cyclic oxazolidine ring prevents the structuring of the growing peptide chain still anchored to the resin and blocks the aggregation of the peptidyl resin and the collapse of the synthesis. The final acid treatment of the peptidyl resin regenerates the Ser or Thr of the native sequence.

The comparison between the second and the third strategy in terms of purity of the end product is observed in the RP-HPLC chromatogram of the thymosin β4 crude product obtained according to a linear SPPS strategy and according to the same Fmoc/tBu strategy but with the use of pseudoprolines is observed. As can be seen in the comparison of both graphs in the linear SPPS strategy, the yield is much lower than in the synthesis object of the present invention.

It must be considered that for conventional linear synthesis, the reactivation in the Ile (34), Thr (33), Lys (31), Ser (30), Pro (29), Leu (28), Pro (27), Asn (26), Lys (16) Ser (15), Lys (14), Asp (13), Phe (12), Lys (11), Glu (10), Ile (9), Glu (8), Ala (7), Met (6), Asp (5), Pro (4), Ser (1) positions was necessary, it being necessary in some cases to recouple: Ile (34), Thr (33), Leu (28), Asn (26), Lys (16), Ser (15), Asp (13), Glu (10), Ile (9), Glu (8), Met (6), Asp (5).

On the contrary, in linear synthesis with pseudoprolines, it was only necessary to reactivate in Ile (34) and Asn (26) and recouple in the latter, which indicates that the addition of pseudoprolines in the synthesis method substantially and surprisingly improves the yield of the method, making it simpler, more advantageous and with a yield that is much greater than the already known methods for synthesizing thymosins.

Once the synthesis object of the present invention has been completed, with or without acetylation of the N-terminal end according to the product, the peptidyl resin is subjected to a final treatment with trifluoroacetic acid and carbocation scavengers to cleave the peptide from the resin and eliminate the protecting groups of the side chains and the pseudoproline protection. Finally, the resulting crude product is purified by RP-HPLC and the group of pure fractions are pooled and lyophilized, the end products being obtained with a purity of more than 99%, with an overall yield greater than 50%.

Table 2 (shown above) shows the difference in yields in these strategies and other previously published strategies for synthesizing β-thymosins. The linear solid-phase synthesis approach according to a Fmoc/tBu strategy without using pseudoprolines gives rise to a crude product that is difficult to purify, the product being obtained with a very low yield (Table 2). However, the use of pseudoproline dipeptides in a linear Fmoc/tBu strategy considerably improves the yield and the purity of the end product. To finish, and as a conclusion of that set forth above, the key and inventive point of the synthesis method claimed in the present invention is based on using pseudoproline dipeptides in key positions of the synthesis, according to the aforementioned guidelines, avoiding sequences causing the aggregation of the growing chain on the resin and thus eliminating incomplete deprotection and coupling problems leading to a low quality peptide crude product and therefore to a low synthesis yield, with the subsequent difficulty in the purification process.

The success of the synthesis process is developed for the present invention as a competitive process for synthesizing thymosins on a large scale, as it as an inexpensive and fast method with a yield and purity greater than those described in the state of the art.

The abbreviations used in the present description have the following meanings:
Ac₂O: Acetic anhydride.
AcOH: Acetic acid.
Boc: Tert-butoxycarbonyl.
DCM: Dichloromethane.
DIEA: N,N'-diisopropylethylamine.
DIPCDI: Diisopropylcarbodiimide.
DMF: N,N-dimethylformamide.
Fmoc: 9-Fluorenylmethoxycarbonyl.
HF: Hydrofluoric acid.
HOBT: N-hydroxybenzotriazole.
HPLC: High Performance Liquid Chromatography.
µL: microliters.
µmol: micromoles.
tBu: Tert-butyl.
TFA: Trifluoroacetic acid.
Trt: trityl.

### Amino acids:

Ala (A): L-Alanine
Arg (R): L-Arginine
Asn (N): L-Asparagine
Ala (A): L-Alanine
Arg (R): L-Arginine
Asn (N): L-Asparagine
Asp (D): L-Aspartic acid
Gln (Q): L-Glutamine
Glu (E): L-Glutamic acid
Gly (G): L-Glycine
Ile (I): L-Isoleucine
Leu (L): L-Leucine
Lys (K): L-Lysine
Met (M): L-Methionine
Phe (F): L-Phenylalanine
Pro (P): L-Proline
Ser (S): L-Serine
Thr (T): L-Threonine
Tyr (Y): L-Tyrosine
Val (V): L-Valine

According to a first important aspect, the present invention relates to a method for obtaining thymosins and/or the pharmaceutically acceptable salts thereof by means of solid-phase synthesis on polymeric supports which comprises the following steps:
a. Linearly synthesizing the thymus-derived peptides in solid phase on a resin, applying the Fmoc/tBu methodology,
b. Incorporating at least one X-Thr and/or X-Ser in the sequence, in pseudoproline dipeptide form,
c. Adding in an intercalated manner the pseudoproline dipeptides with a distance of at least 5 amino acids,
d. Obtaining the thymosins by means of treating the peptidyl resin with trifluoroacetic acid simultaneously causing the breaking of the peptide-resin bond, the deprotection of the side chains and the opening of the pseudoproline cycle,
e. Purifying the thymosins by RP-HPLC.

The present invention relates to a method for obtaining thymosins and/or the pharmaceutically acceptable salts thereof in which α-thymosins and/or the pharmaceutically acceptable salts thereof are obtained.

The present invention relates to a method for obtaining thymosins and/or the pharmaceutically acceptable salts thereof in which β-thymosins and/or the pharmaceutically acceptable salts thereof are obtained.

The present invention relates to a method for obtaining thymosins and/or the pharmaceutically acceptable salts thereof in which the synthesis is carried out on polymeric supports.

The present invention relates to a method for obtaining thymosins and/or the pharmaceutically acceptable salts thereof wherein in step a) a chloro-trityl-type resin is used.

The present invention relates to a method for obtaining thymosins and/or the pharmaceutically acceptable salts thereof wherein in step a) a pMBHA-type resin is used.

The present invention relates to a method for obtaining thymosins and/or the pharmaceutically acceptable salts thereof wherein in step b) the incorporation of Ser or Thr is carried out additionally as pseudoproline dipeptides and/or modified Ser or Thr in pseudoproline form in the peptidyl resin.

The present invention relates to a method for obtaining thymosins and/or the pharmaceutically acceptable salts thereof wherein the minimal separation between two pseudoproline dipeptides or a pseudoproline and a proline is of two amino acids.

The present invention relates to a method for obtaining thymosins and/or the pharmaceutically acceptable salts thereof wherein the pseudoproline dipeptide is preferably added in a hydrophobic amino acid region

The present invention relates to a method for obtaining thymosins and/or the pharmaceutically acceptable salts thereof in which if the Met amino acid appears in the sequence after step d), a detertbutylation treatment of the crude product with AcOH is additionally carried out.

According to a second important aspect, the present invention relates to an isolated and/or purified compound obtained by the previous method comprising an amino acid sequence having at least 80% identity with sequences SEQ.ID.N.1 to SEQ.ID.N.11 over its entire length.

The present invention relates to an isolated and/or purified compound obtained by the previous method consisting of an amino acid sequence chosen from the group consisting of sequences SEQ.ID.N.1 to SEQ.ID.N.11, of the complementary sequences thereof and/or of the homologous sequences thereof and/or of the functional equivalent sequences thereof.

The present invention relates to an isolated and/or purified compound obtained by the previous method including fragments of at least one amino acid sequence chosen from the group consisting of sequences SEQ.ID.N.1 to SEQ.ID.N.11, of the complementary sequences thereof, of the homologous sequences thereof and of the functional equivalent sequences thereof.

The present invention relates to an isolated and/or purified compound obtained by the previous method anterior in which any of the amino acids comprised in sequences SEQ.ID.N.1 to SEQ.ID.N.11 is selected from the group consisting of L-amino acids, D-amino acids, N-methyl amino acids, non-natural amino acids, Met-tBu or oxidized-Met.

The present invention relates to an isolated and/or purified compound obtained by the previous method including modifications in the N-terminal amino acid selected from acylations and/or pegylations.

According to a third important aspect, the present invention relates to the use of the compound obtained by the aforementioned method in the preparation of a medicinal product.

The present invention relates to the use of the compound obtained by the aforementioned method in the preparation of a medicinal product for the regulation of immune response, vascular biology and cancer pathogenesis.

### Embodiments

The following specific examples provided in this patent document are useful for illustrating the nature of the present invention. These examples are solely included for illustrative purposes and must not be interpreted as limitations to the invention claimed in the present specification.

### Example 1: Synthesis of thymosin α1

The Fmoc-Asn-OH C-terminal residue is incorporated on the 2-chlorotrityl resin with a shortage of amino acid for the purpose of obtaining a 0.85 mmol/g functionalization after incorporating the first amino acid. 0.8 g of amino acid (2.13 mmol, 0.5 eq.) are incorporated on 2.5 g of resin (f = 1.6 mmol/g of resin, 4 mmol). The resin and the amino acid are weighed in separate containers and are allowed to dry under vacuum for at least 4 hours. The amino acid is dissolved in 25 mL of dry DCM (on a 4 A sieve). DIEA (0.7 mL, 1.6 mmol, 1 eq.) is added and it is stirred for 5 minutes. A 1:1 solution (2.8 ml) of DIEA:DCM (1.4 mL, 3.2 mmol, 2 eq.) is prepared and is added to the reaction mixture. It is left with stirring for 40 more minutes and then 4 mL of dry MeOH are added and it is allowed to react for 10 minutes, after which the resin is filtered in a synthesis reactor equipped with a filter plate and a key and the following washings are carried out:

| Step | Reagent | Repetitions | Time |
|---|---|---|---|
| 1 | DCM | 3 | 1 min |
| 2 | DMF | 5 | 1 min |
| 3 | 5% piperidine in DMF | 1 | 10 min |
| 4 | 20% piperidine in DMF | 1 | 15 min |
| 5 | DMF | 5 | 1 min |

The next amino acids are incorporated in all the cases with an excess of 2.5 eq of amino acid, HOBT and DIPCDI in relation to the functionalization of the resin after incorporating the first amino acid (0.85 mmol/g). It is allowed to react for 40-60 min and the Fmoc group is subsequently deprotected with 20% of piperidine in DMF for 5 min 2 times and for 10 min another 2 times.

The Fmoc-Ile-Thr(Ψ^{Me,Me}pro)-OH dipeptide (corresponding to amino acids 11-12 of the thymosin α1 sequence) was incorporated using 2.5 eq. of dipeptide, HOBT and DIPCDI, allowing it to react for 1 h.

The coupling efficiency is controlled by means of the ninhydrin test. If it is positive, it is reactivated using 1/3 eq. HOBT and DIPCDI and if it is still positive after the reactivation, it is recoupled using ½ eq. of Fmoc-AA-OH, HOBT and DIPCDI in relation to the equivalents used in the first coupling. If the ninhydrin test is still positive after reactivating, recoupling and reactivating again, acetylation is carried out using 2.5 eq. of Ac₂O, and DIEA for 15 min.

The positions in which it was necessary to reactivate and/or recouple are detailed below: Ser (1), Ser(2), Thr(7), pseudoproline dipeptide (11-12), Lys(14), Leu(16), Lys(17), Glu(18), Lys(19).

The yield at the end of the synthesis is quantitative in obtaining Ac-Ser(tBu)-Asp(OtBu)-Ala-Ala-Val-Asp(OtBu)-Thr(tBu)-Ser(tBu)-Ser(tBu)-Gtu(OtBu)-Ile-Thr(Ψ^{Me,Me}pro)-Thr(tBu)-Lys(Boc)-Asp(OtBu)-Leu-Lys(Boc)-Glu(OtBu)-Lys(Boc)-Lys(Boc)-Glu(OtBu)-Val-Val-Glu(OtBu)-Glu(OtBu)-Ala-Glu(OtBu)-Asn-resin.

7 g of peptidyl resin are treated for 1h 30 min with 80 mL of the TFA: H₂O 95:5 mixture with stirring. Once the reaction has ended, the resin is separated from the product by filtration and washed with TFA. Diethyl ether (560 mL) is added to the TFA solution and the white precipitate is filtered in a P3 plate and washed with MeOH and diethyl ether. Synthesis Yield: 40%.

### Product characterization:

ESI-MS: Theoretical M= 3108 g/mol, Experimental M: (m/z): [M+2H]⁺/2=1555; [M+3H]⁺/3=1037, [M+4H]⁺/4=778, [M+5H]⁺/5=623.
Analytical RP-HPLC: Gradient: 5-85% B in 20 min, tr =11.7 min; Isocratic: 19% B in 30 min, tr =16.5 min (B= 0.07% TFA in acetonitrile).

### Example 2: Synthesis of thymosin β4

Thymosin β4 is synthesized using a solid-phase peptide synthesis strategy using Fmoc/tBu protectors. To incorporate the first amino acid on the 2-chlorotrityl resin (1 g, 1.6 mmol/g), the Nα-Fmoc-protected amino acid (0.5 eq) and DIEA (3 eq.) in DCM are used to achieve a 0.85 mmol/g functionalization after incorporating the first amino acid. It is left with mechanical stirring for 40 min and then 4 mL of dry MeOH are added and it is left to react for 10 min. The resin is filtered in a synthesis reactor equipped with a filter plate and a key and the following washings with DCM and DMF are carried out. The resin is treated with 5% piperidine in DMF 1 x 10 min and 20% piperidine in DMF 1 x 15 min and it is rinsed with DMF. The remaining amino acids are Nα-Fmoc-protected amino acids (3 eq.), HOBT (3 eq.) and DIPCDI (3 eq.) in DMF, the equivalents being calculated in relation to the functionalization of the resin once the first amino acid has been incorporated. The following groups were chosen as protecting groups for the side chains: 2,2,4,6,7-pentamethyldihydrobenzofuran-5-sulfonyl (Pbf) for Arg, tert-butyloxycarbonyl (Boc) for the Lys, Glu and Asp side chains and tert-butyl for the Thr and Ser side chains. The temporary Fmoc protecting group is cleaved by treating with a solution of 20% piperidine in DMF (2 times for 5 min and 2 more times for 10 min). The pseudoproline dipeptides are incorporated in the synthesis as Fmoc-Lys(Boc)-Ser(ΨMe,Me pro)-OH instead of amino acids 14-15 and Fmoc-Glu(OtBu)-Thr(ΨPMe,Me pro)-OH in the positions of amino acids 21-22 and 32-33. Said positions are chosen from the possible positions due to their arrangement in the peptide sequence, optimal proximity to the C-terminal position, optimal proximity to Pro residues and optimal distance between pseudoprolines.

The coupling efficiency is controlled by means of the ninhydrin test. If it is positive, it is reactivated using 1/3 eq. HOBT and DIPCDI and it is still positive after the reactivation, it is recoupled using ½ eq. of Fmoc-AA-OH, HOBT and DIPCDI in relation to the first coupling. If the ninhydrin test is still positive after reactivating, recoupling and reactivating again, acetylation is carried out using 2.5 eq. of Ac₂O, and DIEA for 15 min.

The positions in which it was necessary to reactivate are Ile(34) and Asn(26) and the latter position was also recoupled.

The yield at the end of the synthesis of Ac-Ser(tBu)-Asp(OtBu)-Lys(Boc)-Pro-Asp(OtBu)-Met-Ala-Glu(OtBu)-lle-Glu(OtBu)-Lys(Boc)-Phe-Asp(OtBu)-Lys(Boc)-Ser(Ψ^{Me.Me}pro)-Lys(Boc)-Leu-Lys(Boc)-Lys(Boc)-Thr(tBu)-Glu(OtBu)-Thr(Ψ^{Me.Me}pro)-Gln-Glu(OtBu)-Lys(Boc)-Asn-Pro-Leu-Pro-Ser(tBu)-Lys(Boc)-Glu(OtBu)-Thr(Ψ^{Me.Me}pro)-Ile-Glu(OtBu)-Gln-Glu(OtBu)-Lys(Boc)-Gln-Ala-Gly-Glu(OtBu)-Ser(tBu)-resin is quantitative.

4.8 g of peptidyl resin are treated with 24 mL of the TFA: H₂O 95:5 mixture for 2 h with stirring. The suspension is filtered and the filtrate is precipitated with ether (168 mL). The crude product is filtered in a P3 plate, washed with MeOH and diethyl ether and dried under vacuum. The peptide is treated with a solution 4% AcOH in H₂O for 1 h 30 min at 37°C and is purified by preparative RP-HPLC. The end product is characterized by mass spectrometry in ESI-MS equipment and is analyzed according to specifications. Purity: 99%. Yield: 50%.

### Product characterization:

ESI-MS: Theoretical M = 4962 g/mol, Experimental M: (m/z): [M+4H]⁺/4 = 1242; [M+ 5H]⁺/5 = 994; [M+ 6H]⁺/6 = 828; [M+ 7H]⁺/7 = 710.
Analytical RP-HPLC: Gradient: 5-85% B in 20 min, tr =11.6 min; Isocratic: 20% B in 30 min, tr =19 min (B= 0.07% TFA in acetonitrile).

### Example 3: Synthesis of the thymosin β15

The synthesis method is similar to that used for thymosin β4. The Fmoc-Lys(Boc)-Ser(Ψ^{Me,Me}pro)-OH dipeptide is used in the substitution of amino acids 14-15, Fmoc-Asn(Trt)-Thr(Ψ^{Me,Me}pro)-OH in the positions of amino acids 21-22 and Fmoc-Glu(OtBu)-Thr(Ψ^{Me,Me}pro)-OH in positions 32-33. A coupling was only necessary in Lys(3).

The peptidyl resin Ser(tBu)-Asp(OtBu)-Lys(Boc)-Pro-Asp(OtBu)-Leu-Ser(tBu)-Glu(OtBu)-Val-Glu(OtBu)-Thr(tBu)-Phe-Asp(OtBu)-Lys(Boc)-Ser(Ψ^{Me,Me}pro)-Lys(Boc)-Leu-Lys(Boc)-Lys(Boc)-Thr(tBu)-Asn(Trt)-Thr(Ψ^{Me,Me}pro)-Glu(OtBu)-Glu(OtBu)-Lys(Boc)-Asn-Thr(tBu)-Leu-Pro-Ser(tBu)-Lys(Boc)-Glu(OtBu)-Thr(Ψ^{Me,Me}pro)-Ile-Gln-Gln-Glu(OtBu)-Lys(Boc)-Glu(OtBu)-Tyr(tBu)-Asn-Gln-Arg(Pbf)-Ser(tBu)-resin is obtained with a quantitative yield.

The peptide is cleaved from the resin by reacting it with a TFA:H₂O 95:5 mixture for 1 h 30 min, it is precipitated with diethyl ether and lyophilized. The crude product is purified in a semipreparative RP-HPLC and the end product is characterized by mass spectrometry in ESI-MS equipment. Purity: 98%. Yield: 50%

### Product characterization:

ESI-MS: Theoretical M= 5173 g/mol, Experimental M: (m/z): [M+6H]⁺/6 = 863, [M+7H]⁺/7 = 740, [M+8H]⁺/ 8 = 648, [M+9H]⁺/9 = 576.
Analytical RP-HPLC: Gradient: 5-85% B in 20 min, tr =11.5 min; Isocratic: 20% B in 30 min, tr =11.5 min (B= 0.07% TFA in acetonitrile).

Although the present invention has been described in relation to these particular cases, many other variations and modifications and other uses are related to those specified in the art. The present invention is thus not limited by the specific disclosure herein but only by the claims.

## Claims

1. A method for obtaining thymosins and/or the pharmaceutically acceptable salts thereof by means of solid-phase synthesis on polymeric supports **characterized in that** it comprises the following steps:
a. Linearly synthesizing the thymus-derived peptides in solid phase on a resin, applying the Fmoc/tBu methodology,
b. Incorporating at least one Thr or Ser in the sequence, in pseudoproline dipeptide form,
c. Obtaining the thymosins by means of treating the peptidyl resin with trifluoroacetic acid simultaneously causing the breaking of the peptide-resin bond, the deprotection of the side chains and the opening of the pseudoproline cycle;
d. Purifying the thymosins by RP-HPLC.

2. A method for obtaining thymosins and/or the pharmaceutically acceptable salts thereof according to claim 1, **characterized in that** α-thymosins and/or the pharmaceutically acceptable salts thereof are obtained.

3. A method for obtaining thymosins and/or the pharmaceutically acceptable salts thereof according to claim 1, **characterized in that** β-thymosins and/or the pharmaceutically acceptable salts thereof are obtained.

4. A method for obtaining thymosins and/or the pharmaceutically acceptable salts thereof according to claim 1, **characterized in that** the synthesis is carried out on polymeric supports.

5. A method for obtaining thymosins and/or the pharmaceutically acceptable salts thereof according to claim 1, **characterized in that** in step a) a chloro-trityl-type resin with a 0.1-2 mmol/g functionalization is used.

6. A method for obtaining thymosins and/or the pharmaceutically acceptable salts thereof according to claim 1, **characterized in that** in step a) a pMBHA-type resin with a 0.1-2 mmol/g functionalization is used.

7. A method for obtaining thymosins and/or the pharmaceutically acceptable salts thereof according to claim 1, **characterized in that** in step b) the incorporation of Ser or Thr is carried out in pseudoproline dipeptide form and/or modified Ser or Thr in pseudoproline form in the peptidyl resin.

8. A method for obtaining thymosins and/or the pharmaceutically acceptable salts thereof according to any of claims 1 to 7, **characterized in that** the pseudoproline dipeptide is added in a hydrophobic amino acid region.

9. A method for obtaining thymosins and/or the pharmaceutically acceptable salts thereof according to any of claims 1 to 8, **characterized in that** if the Met amino acid appears in the sequence after step c), a detertbutylation treatment of the crude product with AcOH is additionally carried out.

10. A method according to claim 1, **characterized in that** the thymosins and/or the pharmaceutically acceptable salts thereof comprise an amino acid sequence having at least 80% identity with sequences SEQ.ID.N.1 to SEQ.ID.N.11 over its entire length.

11. A method according to claim 1, **characterized in that** the thymosins and/or the pharmaceutically acceptable salts thereof consist of an amino acid sequence chosen from the group consisting of sequences SEQ.ID.N.1 to SEQ.ID.N.11, and of the complementary sequences thereof.

12. A method according to claim 1, **characterized in that** the thymosins and/or the pharmaceutically acceptable salts thereof include fragments of at least one amino acid sequence chosen from the group consisting of sequences SEQ.ID.N.1 to SEQ.ID.N.11, and of the complementary sequences thereof.

13. A method according to claim 1, **characterized in that** all or any of the amino acids comprised in sequences SEQ.ID.N.1 to SEQ.ID.N.11 are selected from the group consisting of L-amino acids, D-amino acids, N-methyl amino acids, non-natural amino acids, Met-tBu or oxidized-Met.

14. A method according to claim 1, **characterized in that** the thymosins and/or the pharmaceutically acceptable salts thereof include modifications in the N-terminal amino acid selected from acylations and/or pegylations.

15. A method according to any of claim 1 to 14, comprising the additional step of the preparation of a medicinal product comprising said purified thymosins and/or the pharmaceutically acceptable salts thereof.

## Patentansprüche

1. Verfahren zur Erhaltung von Thymosinen und/oder deren pharmazeutisch annehmbaren Salzen durch Festphasensynthese an polymerischen Träger, **dadurch gekennzeichnet, dass** es folgende Schritte umfasst:
a. die lineare Synthese von Thymus abgeleiteter Peptide in fester Phase an einem Harz, unter Anwendung der Fmoc/tBu Methodologie,
b. das Einfügen zumindest eines Thr oder Ser in der Sequenz, in Form eines Pseudoprolin-Dipeptids,
c. das Erhalten des Thymosins durch die Behandlung des Peptidylharzes mit Trifluoressigsäure unter gleichzeitiger Bewirkung der Spaltung der Peptid-Harz-Bindung, der Entschützung der Seitenketten und der Öffnung des Pseudoprolin-Rings;
d. die Reinigung des Thymosins durch RP-HPLC.

2. Verfahren zur Erhaltung von Thymosinen und/oder deren pharmazeutisch annehmbaren Salzen nach Anspruch 1, **dadurch gekennzeichnet, dass** α-Thymosine und/oder deren pharmazeutisch annehmbare Salze erhalten werden.

3. Verfahren zur Erhaltung von Thymosinen und/oder deren pharmazeutisch annehmbaren Salzen nach Anspruch 1, **dadurch gekennzeichnet, dass** β-Thymosine und/oder deren pharmazeutisch annehmbare Salze erhalten werden.

4. Verfahren zur Erhaltung von Thymosinen und/oder deren pharmazeutisch annehmbaren Salzen nach Anspruch 1, **dadurch gekennzeichnet, dass** die Synthese an polymerischen Träger durchgeführt wird.

5. Verfahren zur Erhaltung von Thymosinen und/oder deren pharmazeutisch annehmbaren Salzen nach Anspruch 1, **dadurch gekennzeichnet, dass** im Schritt a) ein Harz des Chlortrityl-Typs mit einer 0,1-2 mmol/g Funktionalisierung verwendet wird.

6. Verfahren zur Erhaltung von Thymosinen und/oder deren pharmazeutisch annehmbaren Salzen nach Anspruch 1, **dadurch gekennzeichnet, dass** im Schritt a) ein Harz des pMBHA-Typs mit einer 0,1-2 mmol/g Funktionalisierung verwendet wird.

7. Verfahren zur Erhaltung von Thymosinen und/oder deren pharmazeutisch annehmbaren Salzen nach Anspruch 1, **dadurch gekennzeichnet, dass** im Schritt b) das Einfügen des Ser oder Thr in Form eines Pseudoprolin-Dipeptids und/oder des modifizierten Ser oder Thr in Form eines Pseudoprolins in das Peptidylharz durchgeführt wird.

8. Verfahren zur Erhaltung von Thymosinen und/oder deren pharmazeutisch annehmbaren Salzen nach einem der Ansprüche 1 bis 7, **dadurch gekennzeichnet, dass** das Pseudoprolin-Dipeptid in einem Bereich hydrophober Aminosäuren zugefügt wird.

9. Verfahren zur Erhaltung von Thymosinen und/oder deren pharmazeutisch annehmbaren Salzen nach einem der Ansprüche 1 bis 8, **dadurch gekennzeichnet, dass** wenn die Aminosäure Met nach Schritt c) in der Sequenz vorhanden ist, zusätzlich eine Detertbutylierungsbehandlung des Rohprodukts mit AcOH durchgeführt wird.

10. Verfahren nach Anspruch 1, **dadurch gekennzeichnet, dass** die Thymosine und/oder deren pharmazeutisch annehmbare Salze eine Aminosäuresequenz umfassen, welche zumindest eine 80%-ige Identität mit den Sequenzen SEQ.ID.N.1 bis SEQ.ID.N.11 über deren gesamte Länge aufweist.

11. Verfahren nach Anspruch 1, **dadurch gekennzeichnet, dass** die Thymosine und/oder deren pharmazeutisch annehmbare Salze aus einer Aminosäuresequenz, ausgewählt aus der Gruppe bestehend aus den Sequenzen SEQ.ID.N.1 bis SEQ.ID.N.11, und deren komplementären Sequenzen bestehen.

12. Verfahren nach Anspruch 1, **dadurch gekennzeichnet, dass** die Thymosine und/oder deren pharmazeutisch annehmbare Salze Fragmente zumindest einer Aminosäuresequenz, ausgewählt aus der Gruppe bestehend aus den Sequenzen SEQ.ID.N.1 bis SEQ.ID.N.11, und deren komplementären Sequenzen beinhalten.

13. Verfahren nach Anspruch 1, **dadurch gekennzeichnet, dass** alle oder jede der in den Sequenzen SEQ.ID.N.1 bis SEQ.ID.N.11 umfassten Aminosäuren aus der Gruppe bestehend aus L-Aminosäuren, D-Aminosäuren, N-Methyl-Aminosäuren, unnatürlichen Aminosäuren, Met-tBu oder oxidiertem Met ausgewählt werden.

14. Verfahren nach Anspruch 1, **dadurch gekennzeichnet, dass** die Thymosine und/oder deren pharmazeutisch annehmbare Salze in der N-endständigen Aminosäure Modifizierungen, ausgewählt aus Acylierungen und/oder Pegylierungen, beinhalten.

15. Verfahren nach einem der Ansprüche 1 bis 14, umfassend den zusätzlichen Schritt der Herstellung eines Arzneimittels, welches die genannte gereinigte Thymosine und/oder deren pharmazeutisch annehmbare Salze umfasst.

## Revendications

1. Procédé pour obtenir des thymosines et/ou leurs sels pharmaceutiquement acceptables au moyen de synthèse en phase solide sur supports polymériques **caractérisé en ce qu'**il comprend les étapes suivantes :
a. synthétiser linéairement les peptides dérivés de thymus en phase solide sur une résine, en appliquant la méthodologie Fmoc/tBu,
b. incorporer au moins un Thr ou Ser dans la séquence, sous forme de dipeptide pseudoproline,
c. obtenir les thymosines au moyen de traitement de la résine de peptidyle avec de l'acide trifluoroacétique en causant simultanément la rupture de la liaison peptide-résine, la déprotection des chaînes latérales et l'ouverture du cycle pseudoproline ;
d. purifier les thymosines par RP-HPLC.

2. Procédé pour obtenir des thymosines et/ou leurs sels pharmaceutiquement acceptables selon la revendication 1, **caractérisé en ce que** l'on obtient des α-thymosines et/ou leurs sels pharmaceutiquement acceptables.

3. Procédé pour obtenir des thymosines et/ou leurs sels pharmaceutiquement acceptables selon la revendication 1, **caractérisé en ce que** l'on obtient des β-thymosines et/ou leurs sels pharmaceutiquement acceptables.

4. Procédé pour obtenir des thymosines et/ou leurs sels pharmaceutiquement acceptables selon la revendication 1, **caractérisé en ce que** la synthèse est réalisée sur des supports polymériques.

5. Procédé pour obtenir des thymosines et/ou leurs sels pharmaceutiquement acceptables selon la revendication 1, **caractérisé en ce que** dans l'étape a) on utilise une résine de type chloro-trityle avec une fonctionnalisation de 0,1-2 mmol/g.

6. Procédé pour obtenir des thymosines et/ou leurs sels pharmaceutiquement acceptables selon la revendication 1, **caractérisé en ce que** dans l'étape a) on utilise une résine de type pMBHA avec une fonctionnalisation de 0,1-2 mmol/g.

7. Procédé pour obtenir des thymosines et/ou leurs sels pharmaceutiquement acceptables selon la revendication 1, **caractérisé en ce que** dans l'étape b) l'incorporation de Ser ou Thr est réalisée sous forme de dipeptide pseudoproline et/ou Ser ou Thr modifié sous forme de pseudoproline dans la résine de peptidyle.

8. Procédé pour obtenir des thymosines et/ou leurs sels pharmaceutiquement acceptables selon l'une quelconque des revendications 1 à 7, **caractérisé en ce que** le dipeptide pseudoproline est ajouté dans une région d'acide aminé hydrophobe.

9. Procédé pour obtenir des thymosines et/ou leurs sels pharmaceutiquement acceptables selon l'une quelconque des revendications 1 à 8, **caractérisé en ce que** si l'aminoacide Met apparaît dans la séquence après l'étape c), on réalise en outre un traitement de déterbutylation des produits bruts avec AcOH.

10. Procédé selon la revendication 1, **caractérisé en ce que** les thymosines et/ou leurs sels pharmaceutiquement acceptables comprennent une séquence aminoacide ayant au moins 80% d'identité avec les séquences SEQ.ID.N.1 à SEQ.ID.N.11 sur toute sa longueur.

11. Procédé selon la revendication 1, **caractérisé en ce que** les thymosines et/ou leurs sels pharmaceutiquement acceptables consistent en une séquence aminoacide choisie parmi le groupe consistant aux séquences SEQ.ID.N.1 à SEQ.ID.N.11, et à leurs séquences complémentaires.

12. Procédé selon la revendication 1, **caractérisé en ce que** les thymosines et/ou leurs sels pharmaceutiquement acceptables comportent des fragments d'au moins une séquence aminoacide choisie parmi le groupe consistant aux séquences SEQ.ID.N.1 à SEQ.ID.N.11, et à leurs séquences complémentaires.

13. Procédé selon la revendication 1, **caractérisé en ce que** tous ou l'un quelconque des aminoacides compris dans les séquences SEQ.ID.N.1 à SEQ.ID.N.11 sont choisis parmi le groupe consistant en L-aminoacides, D-aminoacides, aminoacides N-méthylés, aminoacides non naturels, Met-tBu ou Met oxydé

14. Procédé selon la revendication 1, **caractérisé en ce que** les thymosines et/ou leurs sels pharmaceutiquement acceptables comportent des modifications dans l'aminoacide n-terminal choisies à partir d'acylations et/ou pégylations.

15. Procédé selon l'une quelconque des revendications 1 à 14, comprenant l'étape additionnelle de la préparation d'un produit médicinal comprenant lesdites thymosines purifiées et/ou leurs sels pharmaceutiquement acceptables.


### SEQUENCE LISTING

<110> BCN-PEPTIDES
<120> METHOD FOR SYNTHESIZING THYMOSINS
<130> TIMOSINAS-BCN
<140> ES-2006-02-27
   <141> 2006-04-24
<160> 11
<170> PatentIn version 3.3
<210> 1
   <211> 28
   <212> PRT
   <213> Artificial
<220>
   <223> Thymosin-alpha-1
<400> 1
   Ser Asp Ala Ala Val Asp Thr Ser Ser Glu Ile Thr Thr Lys Asp Leu
   1               5                   10                  15
   Lys Glu Lys Lys Glu Val Val Glu Glu Ala Glu Asn
               20                  25
<210> 2
   <211> 24
   <212> PRT
   <213> Artificial
<220>
   <223> Des-(25-28)-Thymosin-alpha-1
<400> 2
   Ser Asp Ala Ala Val Asp Thr Ser Ser Glu Ile Thr Thr Lys Asp Leu
   1               5                   10                  15
   Lys Glu Lys Lys Glu Val Val Glu
               20
<210> 3
   <211> 35
   <212> PRT
   <213> Artificial
<220>
   <223> Thymosin-alpha-11
<400> 3
   Ser Asp Ala Ala Val Asp Thr Ser Ser Glu Ile Thr Thr Lys Asp Leu
   1               5                   10                  15
   Lys Glu Lys Lys Glu Val Val Glu Glu Ala Glu Asn Gly Arg Glu Ala
               20                  25                  30
   Pro Ala Asn
           35
<210> 4
   <211> 43
   <212> PRT
   <213> Artificial
<220>
   <223> Thymosin-beta-4
<400> 4
   Ser Asp Lys Pro Asp Met Ala Glu Ile Glu Lys Phe Asp Lys Ser Lys
   1 5 10 15
   Leu Lys Lys Thr Glu Thr Gln Glu Lys Asn Pro Leu Pro Ser Lys Glu
   20 25 30
   Thr Ile Glu Gln Glu Lys Gln Ala Gly Glu Ser
   35 40
<210> 5
   <211> 44
   <212> PRT
   <213> Artificial
<220>
   <223> Thymosin-Beta-4Xen
<400> 5
   Ser Asp Lys Pro Asp Met Ala Glu Ile Glu Lys Phe Asp Lys Ala Lys
   1 5 10 15
   Leu Lys Lys Thr Glu Thr Gln Glu Lys Asn Pro Leu Pro Ser Lys Glu
   20 25 30
   Thr Ile Glu Asn Glu Lys Asn Thr Ser Gly Glu Ser
   35 40
<210> 6
   <211> 39
   <212> PRT
   <213> Artificial
<220>
   <223> Thymosin-Beta-8
<400> 6
   Ala Asp Lys Pro Asp Leu Gly Glu Ile Asn Ser Phe Asp Lys Ala Lys
   1 5 10 15
   Leu Lys Lys Thr Glu Thr Gln Glu Lys Asn Thr Leu Pro Thr Lys Glu
   20 25 30
   Thr Ile Glu Gln Glu Lys Gln
   35
<210> 7
   <211> 41
   <212> PRT
   <213> Artificial
<220>
   <223> Thymosin-Beta-9
<400> 7
   Ala Asp Lys Pro Asp Leu Gly Glu Ile Asn Ser Phe Asp Lys Ala Lys
   1               5                   10                  15
   Leu Lys Lys Thr Glu Thr Gln Glu Lys Asn Thr Leu Pro Thr Lys Glu
               20                  25                  30
   Thr Ile Glu Gln Glu Lys Gln Ala Lys
           35                  40
<210> 8
   <211> 43
   <212> PRT
   <213> Artificial
<220>
   <223> Thymosin-Beta-10
<400> 8
   Ala Asp Lys Pro Asp Met Gly Glu Ile Ala Ser Phe Asp Lys Ala Lys
   1               5                   10                  15
   Leu Lys Lys Thr Glu Thr Gln Glu Lys Asn Thr Leu Pro Thr Lys Glu
               20                  25                  30
   Thr Ile Glu Gln Glu Lys Arg Ser Glu Ile Ser
           35                  40
<210> 9
   <211> 41
   <212> PRT
   <213> Artificial
<220>
   <223> Thymosin-Beta-11
<400> 9
   Ser Asp Lys Pro Asn Leu Glu Glu Val Ala Ser Phe Asp Lys Thr Lys
   1               5                   10                  15
   Leu Lys Lys Thr Glu Thr Gln Glu Lys Asn Pro Leu Pro Thr Lys Glu
               20                  25                  30
   Thr Ile Glu Gln Glu Lys Gln Ala Ser
           35                  40
<210> 10
   <211> 42
   <212> PRT
   <213> Artificial
<220>
   <223> Thymosin-Beta-12
<400> 10
   Ser Asp Lys Pro Asp Leu Ala Glu Val Ser Asn Phe Asp Lys Thr Lys
   1               5                   10                  15
   Leu Lys Lys Thr Glu Thr Gln Glu Lys Met Pro Leu Pro Thr Lys Glu
               20                  25                  30
   Thr Ile Glu Gln Glu Lys Gln Ala Thr Ala
           35                  40
<210> 11
   <211> 44
   <212> PRT
   <213> Artificial
<220>
   <223> Thymosin-Beta-15
<400> 11
   Ser Asp Lys Pro Asp Leu Ser Glu Val Glu Thr Phe Asp Lys Ser Lys
   1               5                   10                  15
   Leu Lys Lys Thr Asn Thr Glu Glu Lys Asn Thr Leu Pro Ser Lys Glu
               20                  25                  30
   Thr Ile Gln Gln Glu Lys Glu Tyr Asn Gln Arg Ser
           35                  40